Europäisches Patentamt

(19) European Patent Office

Office européen des brevets

(11) Numéro de publication: **0 195 719**
**B1**

(12) **FASCICULE DE BREVET EUROPEEN**

(45) Date de publication du fascicule du brevet:
13.09.89

(51) Int. Cl.⁴: **C07C 17/24**
// C07C21/06, C07C17/34

(21) Numéro de dépôt: 86400571.5

(22) Date de dépôt: 18.03.86

(54) Procédé continu de craquage de dichloro-1,2 éthane.

(30) Priorité: 20.03.85 FR 8504101

(43) Date de publication de la demande:
24.09.86 Bulletin 86/39

(45) Mention de la délivrance du brevet:
13.09.89 Bulletin 89/37

(84) Etats contractants désignés:
AT BE CH DE FR GB IT LI LU NL SE

(56) Documents cités:
DE-B- 1 210 800
FR-A- 1 306 945

KIRK-OTHMER, Encyclopedia of Chemical Technology,
Third Edition, V. 12, P.984-991

(73) Titulaire: ATOCHEM, 4 & 8, Cours Michelet La
Défense 10, F-92800 Puteaux(FR)

(72) Inventeur: Demaiziere, Claude, 33 Boulevard des
Esterelles, F-13500 Martigues(FR)
Inventeur: Lesparre, Jean, Quartier Savin,
F-04290 Volonne(FR)
Inventeur: Correia, Yves, Les Lauzières,
F-04160 Chateau-Arnoux(FR)

(74) Mandataire: Rochet, Michel et al, ATOCHEM
Département Propriété Industrielle La
Défense 10 4 & 8 Cours Michelet, F-92800 Puteaux(FR)

ACTORUM AG

## Description

L'invention a pour objet un procédé continu de craquage du dichloro-1,2 éthane en chlorure de vinyle.

Les procédés de craquage consistent à porter les vapeurs de dichloro-1,2 éthane à une température de l'ordre de 300 à 650°C et sous une pression de 8 à 40 bars. Pour limiter le taux de produits lourds formés au cours de cette opération (hydrocarbures chlorés ayant au moins 4 atomes de carbone et pouvant générer des particules charbonneuses pouvant encrasser le four de craquage), le taux de conversion (rapport molaire dichloro-1,2 éthane craqué/dichloro-1,2 éthane introduit) peut être compris entre 50 et 70% (brevet français 1 302 458) et plus précisément entre 55 et 65% (brevet français 1 491 946). En fait en exploitation industrielle ce taux est habituellement limité à 55% (Techniques de l'Ingénieur 9–1984).

Dans le brevet français 721 808 on a proposé de réaliser la réaction de craquage en présence de vapeur d'eau ou d'un diluant tel que l'acide chlorhydrique, l'azote ou l'anhydride carbonique. Selon ce brevet, qui vise à l'obtention simultanée de chlorure de vinyle et d'acétylène, on opère à une température d'au moins 800°C et de préférence comprise entre 800 et 1000°C, le diluant recommandé étant la vapeur d'eau.

Le brevet français 1 306 945 propose de limiter le taux de conversion ou, si on souhaite avoir un taux de conversion élevé, d'ajouter un diluant tel que l'acide chlorhydrique. Le procédé décrit dans ce brevet français 1 306 945 concerne cependant, et exclusivement un procédé de dissociation catalytique du dichloro-1,2 éthane. Ce procédé, qui fait appel à un catalyseur solide, est généralement mis en œuvre à une température comprise entre 160 et 350°C, et sous une pression absolue n'excédant pas 2,5 atmosphères. Selon ce procédé la diminution de la concentration du chlorure de vinyle s'obtient par addition du même volume d'acide chlorhydrique.

Le brevet allemand DE-AS 1 210 800 décrit un procédé de craquage thermique du dichloro-éthane en présence d'halogène ou d'un produit pouvant générer des halogènes. Ce procédé concerne surtout l'addition de chlore ou d'un produit chloré à 2 atomes de carbone au dichloro-éthane avant craquage.

L'invention propose un procédé de craquage thermique, pouvant être en œuvre à une température très inférieure à 800°C et dans lequel le taux de conversion peut atteindre 90% voire davantage sans danger pour l'appareillage ou la rentabilité du procédé.

Le procédé conforme à l'invention est un procédé de craquage thermique du dichloro-1,2 éthane, effectué à une température comprise entre 300 et 650°C et sous une pression absolue comprise entre 1 et 40 bars, ledit procédé étant caractérisé en ce que la réaction s'effectue en présence d'acide chlorhydrique.

L'invention concerne plus précisément un procédé tel que défini ci-avant, dans lequel le rapport molaire acide chlorhydrique/dichloro-1,2 éthane est compris entre 0,1 et 1,8 et de préférence est compris entre 0,2 et 1,5. Ce procédé est de préférence mis en oeuvre à une température comprise entre 350 et 550°C et/ou sous une pression absolue comprise entre 1 et 25 bars.

Selon une variante de ce procédé, on ajoute au flux entrant dans le four (dichloro-1,2 éthane et HCl) un ou plusieurs produits susceptibles de donner des radicaux libres tels que $Cl_2$, $CCl_4$, $C_2Cl_6$ sans que cette liste soit limitative. D'une manière générale, lorsqu'on fait appel à de tels additifs, le rapport molaire additif/dichloro-1,2 éthane peut atteindre $10^{-2}$ et de préférence est compris entre $10^{-4}$ et $5.10^{-3}$.

Ce procédé peut être réalisé dans l'appareillage généralement utilisé pour le craquage et notamment dans les fours multitubulaires dans lesquels l'efficacité de ce procédé est particulièrement visible compte tenu de la sensibilité de ces réacteurs à l'encrassement.

La mise en oeuvre de ce procédé permet de travailler avec un taux de conversion très élevé, pouvant dépasser 95 %. Il est remarquable de constater que, malgré un tel taux de conversion, les fours peuvent être utilisés pendant de très longues durées sans observer de dépôt charbonneux sur la paroi de ces fours.

Les exemples suivant illustrent l'invention.

Dans ces exemples, D 12 signifie dichloro-1,2 éthane et CV désigne le chlorure de vinyle.

### EXEMPLE 1:

Dans un réacteur constitué d'un serpentin en inconel de 8 mm de diamètre intérieur et de 3,48 m de long, maintenu à 480°C, par un bain de plomb, on introduit 2,5 moles/heure de D 12 et 1,61 mole/heure d'HCl. La pression dans le réacteur est la pression atmosphérique. Le flux sortant du réacteur, après absorption d'HCl, a la composition du tableau ci-après. Le taux de craquage ou de conversion du D 12 est de 97,5 % molaire.

A titre d'exemple comparatif, et dans le but d'obtenir la même teneur en chlorure de vinyle de l'évent gazeux, on procède à un essai où l'on introduit dans les mêmes conditions de température et pression que pour l'essai ci-dessus, 5,77 moles/heure de D 12 ; le flux sortant du réacteur a la composition donnée en annexe, le taux de conversion du D 12 est de 56,2 molaires. On note le fort débit de "solvants condensés" constitués essentiellement de D 12 non craqué.

On note qu'après 10 heures de marche continue, le fonctionnement du procédé selon l'invention est toujours correct. Dans un essai comparatif effectué avec 3 moles/heure de D 12 pour réaliser un taux de craquage de 96-98 % le four est complètement obturé par les lourds au bout d'une heure de marche.

EP 0 195 719 B1

| | | Exemple 1 | Contre-essai |
|---|---|---|---|
| Event gazeux Composition | Débit (mole/heure) | 2,44 | 3.24 |
| $C_2H_2$ (% mole) | | 0,0635 | 0,2370 |
| $C_2H_4$ (% mole) | | 0,0215 | 0,0320 |
| CV (% mole) | | 98,3950 | 98,6580 |
| Chloroprènes | | 0,2750 | – |
| $CHCl_3$ (% mole) | | 0,8774 | 1,0730 |
| Solvants condensés Composition | Débit (g/h) | 15,4 | 231,7 |
| Impuretés légères* (% poids) | | 1,77 | 1,03 |
| D 12 (% poids) | | 93,6510 | 93,3610 |
| Impuretés lourdes** (% poids) | | 1,56 | 0,14 |

\* Légers = trichloro-1,1,1 éthane, $CHCl_3$, chloroprène, $CCl_4$, trichoréthylène notamment
\*\* Lourds = dérivés chlorés $C_3$, $C_4$, $C_6$, notamment

## EXEMPLE 2 à 6:

On renouvelle l'essai de l'exemple 1 en suivant les conditions opératoires et en observant les résultats rassemblés dans le tableau qui suit :

| Exemples | 2 | 3 | 4 | 5 | 6 |
|---|---|---|---|---|---|
| Température four °C | 490 | 470 | 450 | 490 | 490 |
| Alimentation: | | | | | |
| · D 12 (mole/h) | 2,4 | 5 | 4 | 4,35 | 4,35 |
| · HCl (mole/h) | 3,6 | 7,5 | 6 | 1,65 | 1,65 |
| · $Cl_2$ (mg/kg D 12) | – | 5200 | 5600 | 5200 | 1580 |
| Taux de craquage ex D 12 (%) | 75,1 | 85,4 | 85,7 | 90,5 | 80,5 |
| Impuretés légères (% du poids du CV) | 1,55 | 1,65 | 1,45 | 1,61 | 1,90 |
| Impuretés lourdes (% du poids du CV) | 0,49 | 0,60 | 1,09 | 0,68 | 0,56 |

Le D 12 utilisé est un D 12 provenant d'une unité industrielle renfermant 99,5 % en poids de dichloro-1,2 éthane.

## EXEMPLE 7:

Dans un four industriel multitubulaire fonctionnant sous 12 bars (pression absolue) avec une température de sortie de 500°C, on effectue une opération de craquage de D 12 dans les conditions et avec les résultats suivants :

| | |
|---|---|
| – Aliméntation: | |
| · D 12 (K.mole/h) | 276,6 |
| · HCl (K.mole/h) | 147,7 |
| · Initiateur | $10^{-3}$ mole/mole de D 12 |
| – Taux de craquage: | |
| · ex D 12 (%) | 82,6 |
| – Sélectivité en chlorure de vinyle | 99,23% |

A titre de comparaison, pour obtenir la même production horaire de chlorure de vinyle, dans une installation fonctionnant avec le même taux d'initiateur mais sans HCl, il faut alimenter le four avec 415,4 K.mole/h de D 12 (taux de conversion 55 % avec une sélectivité de 99,3 %).

3

**Revendications**

1. Procédé de préparation du chlorure de vinyle par craquage thermique du dichloro-1,2 éthane, effectué à une température comprise entre 300 et 650°C et sous une pression absolue comprise entre 1 et 40 bars, ledit procédé étant caractérisé en ce que la réaction s'effectue en présence d'acide chlorhydrique et que le rapport molaire acide chlorhydrique/dichloro-1,2 éthane est compris entre 0,1 et 1,8.

2. Procédé selon la revendication 1, caractérisé en ce que le rapport molaire acide chlorhydrique/dichloro-1,2 éthane est compris entre 0,2 et 1,5.

3. Procédé selon l'une quelconque des revendications 1 ou 2, caractérisé en ce qu'on ajoute au dichloro-1,2 éthane et à l'acide chlorhydrique des produits susceptibles de donner des radicaux libres.

4. Procédé selon la revendication 4, caractérisé en ce que les produits susceptibles de donner des radicaux libres sont choisis dans le groupe formé par $Cl_2$, $CCl_4$, $C_2Cl_6$.

5. Procédé selon l'une quelconque des revendications 3 ou 4, caractérisé en ce que le rapport molaire additif/dichloro-1,2 éthane peut atteindre $10^{-2}$ et de préférence est compris entre $10^{-4}$ et $5 \cdot 10^{-3}$.

**Patentansprüche**

1. Verfahren zur Herstellung von Vinylchlorid durch thermisches Cracken von 1,2-Dichlorethan bei einer Temperatur von 300 bis 650°C und einem Druck von 1 bis 40 bar abs., dadurch gekennzeichnet, daß die Zersetzung in Gegenwart von Chlorwasserstoff stattfindet und das Molverhältnis Chlorwasserstoff zu Dichlorethan 0,1 bis 1,8 beträgt.

2. Verfahren nach Anspruch 1, dadurch gekennzeichnet, daß das Molverhältnis Chlorwasserstoff zu Dichlorethan 0,2 bis 1,5 beträgt.

3. Verfahren nach Anspruch 1 oder 2, dadurch gekennzeichnet, daß man zusätzlich einen Radikalbildner verwendet.

4. Verfahren nach Anspruch 3, dadurch gekennzeichnet, daß man als Radikalbildner $Cl_2$, $CCl_4$ und/oder $C_2Cl_6$ verwendet.

5. Verfahren nach Anspruch 3 oder 4, dadurch gekennzeichnet, daß das Molverhältnis Radikalbildner zu Dichlorethan bis $10^{-2}$, vorzugsweise $10^{-4}$ bis $5 \cdot 10^{-3}$ beträgt.

**Claims**

1. Process for the preparation of vinyl chloride by thermal cracking of 1,2-dichloroethane, carried out at a temperature of between 300 and 650°C and an absolute pressure of between 1 and 40 bars, the said process being characterized in that the reaction is carried out in the presence of hydrochloric acid and that the molar ratio hydrochloric acid/1,2-dichloroethane is between 0.1 and 1.8.

2. Process according to claim 1, characterized in that the molar ratio hydrochloric acid/1,2-dichloroethane is between 0.2 and 1.5.

3. Process according to either of claims 1 and 2, characterized in that substances capable of giving free radicals are added to 1,2-dichloroethane and to hydrochloric acid.

4. Process according to claim 4, characterized in that the substances capable of giving free radicals are chosen from the group consisting of $Cl_2$, $CCl_4$ and $C_2Cl_6$.

5. Process according to either of claims 3 and 4, characterized in that the molar ratio additive/1,2-dichloroethane can reach $10^{-2}$ and is preferably between $10^{-4}$ and $5 \times 10^{-3}$.